# EUROPEAN PATENT APPLICATION

(11) **EP 4 464 253 A1**
(43) Date of publication of application: **20.11.2024**
(21) Application number: 23173567.1
(22) Date of filing: 16.05.2023
(51) Int. Cl.: A61B 6/03, A61B 6/00, H05G 1/08, H05G 1/54

(54) **FAULT IDENTIFICATION IN MEDICAL IMAGING SYSTEM ELECTRICAL CIRCUITS**

(71) Applicant: Koninklijke Philips N.V., 5656 AG Eindhoven (NL)
(72) Inventor: LEUSSLER, Christoph Günther, Eindhoven (NL); LIPS, Oliver, Eindhoven (NL); VOGTMEIER, Gereon, 5656AG Eindhoven (NL); PREVRHAL, Sven Peter, Eindhoven (NL)
(74) Representative: Philips Intellectual Property & Standards

(57) **Abstract**

A system (100) for identifying a potential fault in an electrical circuit (110) comprising a component (120) of a medical imaging system (130) and a switching power supply (140) configured to supply power to the component, is provided. The system includes a receiver circuit (150, and an analyser circuit (160). The receiver circuit (150) receives radiofrequency, RF, signals generated by the switching power supply (140) during operation of the electrical circuit (110). The analyser circuit (160) determines, based on the received RF signals, an amplitude and/or a phase of one or more harmonic frequencies of a switching frequency of the switching power supply (140). The analyser circuit (160) is identifies the potential fault in the electrical circuit (110) based on the determined amplitude and/or phase of the one or more harmonic frequencies.

## Description

### FIELD OF THE INVENTION

The present disclosure relates generally to the identification of faults in electrical circuits of medical imaging systems. More specifically, it relates to the identification of a potential fault in an electrical circuit that includes a component of a medical imaging system and a switching power supply. A system, a medical imaging system that includes the system, a computer-implemented method, and a computer program product, are disclosed.

### BACKGROUND OF THE INVENTION

Medical imaging systems such as projection X-ray imaging systems, computed tomography, "CT", imaging systems, magnetic resonance imaging, "MRI", systems, and so forth, include numerous electrical circuits. The electrical circuits in such medical imaging systems typically include a power supply, and one or more components. For instance, an example of an electrical circuit in a CT imaging system is the electrical circuit that includes a power supply in the form of a so-called "high voltage generator", or simply a "generator", and a component in the form of an X-ray tube.

One type of power supply that is used to supply power to the components of medical imaging systems is a switching power supply. A switching power supply is also known as a switched-mode power supply, "SMPS", a switching-mode power supply, a switch-mode power supply, a switched power supply, or simply as a "switcher". Various topologies of switching power supplies are known, including buck, boost, and buck-boost, also known as an inverter topology. Switching power supplies exploit a common principle wherein a current is switched into a load via one or more non-linear circuit elements, such as diodes, in order to provide a desired output voltage across the load.

Switching power supplies often include a switching element, e.g. a metal oxide semiconductor field effect transistor "MOSFET" switch, that switches the current into the load via the one or more non-linear circuit elements. The switching element in this category of switching power supply is typically switched between the fully-on and the fully-off states. This is in contrast to a linear power supply, and in which a switching element delivers a current to a load by maintaining the switching element in a partially-on state, and which dissipates heat in the switching element.

Some switching power supplies do not include a switching element. Switching power supplies in this category are instead supplied by an alternating current. The alternating current is similarly supplied to a load via one or more non-linear circuit elements, and the current is inherently switched due to its alternating nature. Examples of switching power supplies in this category include voltage multipliers, such as the Greinacher multiplier, also known as the Villard cascade, and the Cockcroft-Walton generator. Such circuits may be used to convert a relatively lower AC voltage into a relatively higher DC voltage.

In switching power supplies, current is switched at a so-called switching frequency. In some categories of switching power supplies the switching frequency is fixed, and in other categories the switching frequency is variable. In the former category, the duty cycle of the switching current is varied in order to deliver a desired amount of power to the load. In the latter category, a frequency, of the switching element is controlled in order to deliver a desired amount of power to the load. The switching frequency of switching power supplies is often in the range from approximately 1kHz to a few megahertz.

Occasionally, faults may develop in the electrical circuits of medical imaging systems. Faults may occur as a result of degradation, or as a result of a failure of circuit elements in the electrical circuit. For instance, in an electrical circuit in a CT imaging system that includes a high voltage generator and an X-ray tube, the X-ray tube may become degraded, or even fail, as a result of arcing. Similarly, other circuit elements such as electrical cables, or connectors, that couple the high voltage generator to the component, and also circuit elements within the high voltage generator itself, may become degraded, or they may fail. Such faults can impact the operation of the medical imaging system. In the case of a minor fault, the impact may not be too severe. For instance, the quality of medical images generated by the medical imaging system may be degraded. In the case of a more severe fault, imaging procedures may need to be suspended. In either case, workflow may be interrupted whilst the cause of the fault has been diagnosed and the fault rectified.

Numerous monitoring procedures are performed on medical imaging systems in order to identify such faults. The monitoring data from such procedures is typically stored in log files which are analysed pro-actively, and also reactively, in order to identify potential future faults, and to identify faults that have already occurred. However, many faults remain difficult to identify. This is in-part due to the complexity of the electrical circuits in medical imaging systems.

Consequently, there remains a need to improve the identification of faults in the electrical circuits of medical imaging systems.

### SUMMARY OF THE INVENTION

According to one aspect of the present disclosure, a system for identifying a potential fault in an electrical circuit comprising a component of a medical imaging system and a switching power supply configured to supply power to the component, is provided. The system includes:
- a receiver circuit configured to receive radiofrequency, RF, signals generated by the switching power supply during operation of the electrical circuit; and
- an analyser circuit configured to determine, based on the received RF signals, an amplitude and/or a phase of one or more harmonic frequencies of a switching frequency of the switching power supply, and which one or more harmonic frequencies are generated by the switching power supply in response to a loading of the switching power supply by the component.

The analyser circuit is further configured to identify the potential fault in the electrical circuit based on the determined amplitude and/or phase of the one or more harmonic frequencies.

An insight exploited by the inventors of the above system is that a switching power supply generates harmonic frequencies, i.e. signals at integer multiples of its switching frequency, that have amplitude, and also phase, values, that are dependent upon the electrical load that is presented to the power supply. In the above system, a load is presented to the switching power supply by the component. The value of the load is determined by the electrical impedance of the component. As a result, the switching power supply generates a set of harmonic frequencies with amplitude and/or phase values that are characteristic of the component.

A further insight of the inventors that is exploited in the above system is that a fault in a component affects its impedance. In the above system, a change in the impedance of the component, changes the loading of the switching power supply. The change in the loading of the switching power supply, changes the amplitude and/or phase values of the harmonic frequencies generated by the switching power supply. Similarly, faults that occur in circuit elements that are elsewhere in the electrical circuit in the above system, also affect their impedance, resulting in changes in the amplitude and/or phase values of the harmonic frequencies generated by the switching power supply. For instance, faults in electrical connectors, or in cables, that couple the switching power supply to the component, and also faults in circuit elements within the switching power supply itself, also result in changes in the amplitude and/or phase values of the harmonic frequencies generated by the switching power supply. Consequently, it is possible to identify potential faults in the electrical circuit that is formed by a switching power supply, and a component of a medical imaging system, based on an amplitude and/or a phase of the one or more harmonic frequencies that are generated by the switching power supply in response to a loading of the switching power supply by the component.

Further aspects, features, and advantages of the present disclosure will become apparent from the following description of examples, which is made with reference to the accompanying drawings.

### BRIEF DESCRIPTION OF THE DRAWINGS

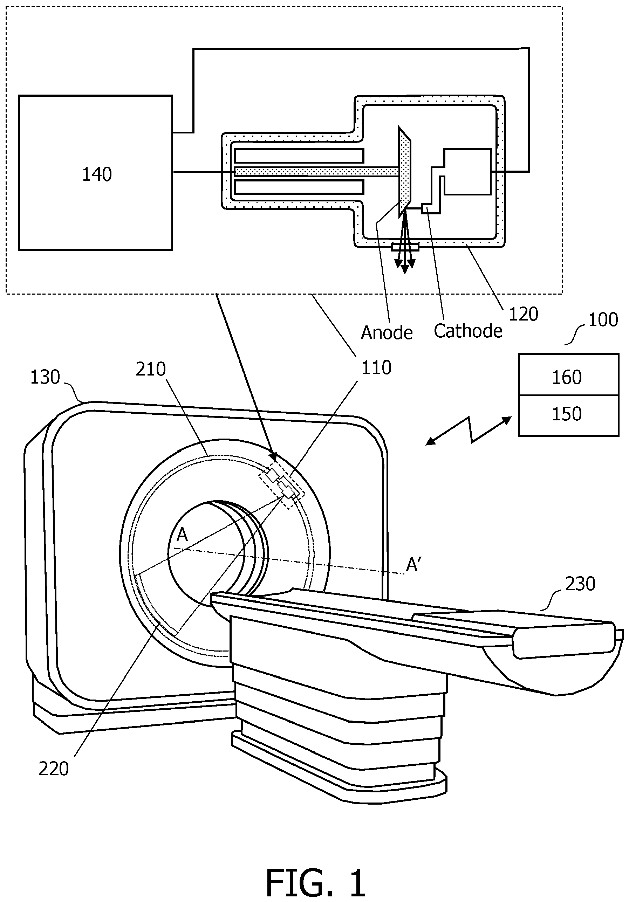

Fig. 1 is a schematic diagram illustrating an example of a system 100 for identifying a potential fault in an electrical circuit 110, in accordance with some aspects of the present disclosure.

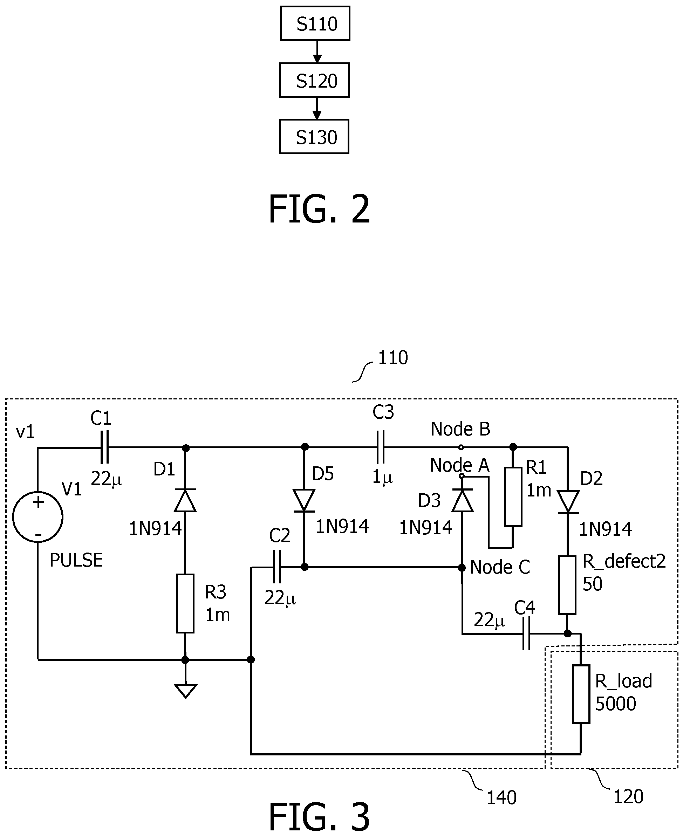

Fig. 2 is a flowchart illustrating an example of a method of identifying a potential fault in an electrical circuit, in accordance with some aspects of the present disclosure.
Fig. 3 is an example of a circuit diagram of an electrical circuit 110 including a switching power supply 140 and a component 120 of a medical imaging system, for use by an electrical circuit simulator to generate simulated amplitude and simulated phase values, in accordance with some aspects of the present disclosure.

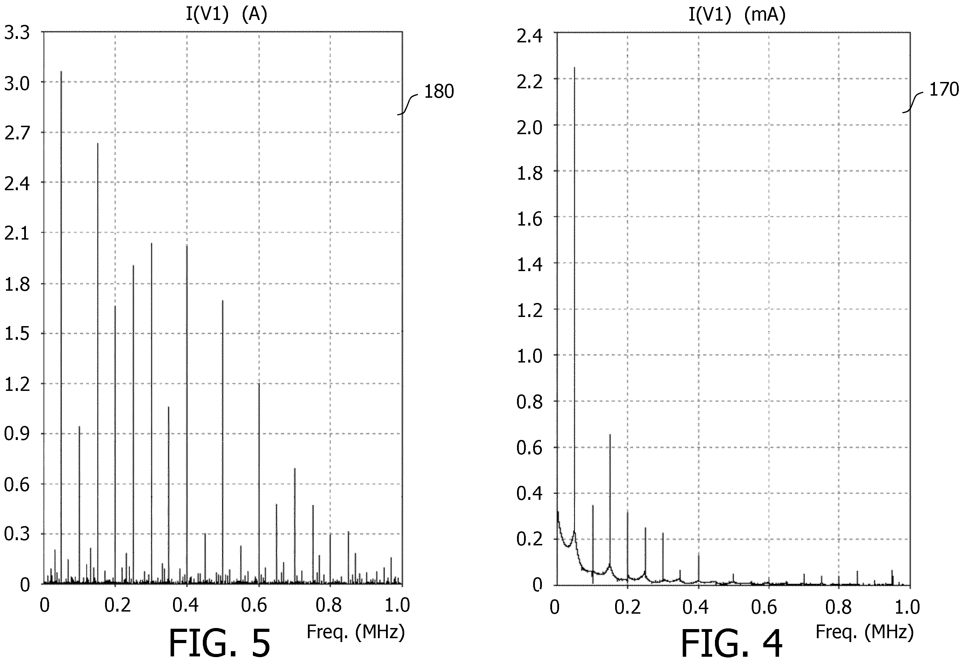

Fig. 4 is an example of a simulation result obtained by simulating an electrical circuit 110 including a switching power supply and a component of a medical imaging system, in the absence of a simulated defect in an electrical component of the switching power supply, in accordance with some aspects of the present disclosure.
Fig. 5 is an example of a simulation result obtained by simulating an electrical circuit 110 including a switching power supply and a component of a medical imaging system, in the presence of a simulated defect in an electrical component of the switching power supply, in accordance with some aspects of the present disclosure.

### DETAILED DESCRIPTION OF EMBODIMENTS

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a system, may be implemented in a computer implemented method, and in a computer program product, in a corresponding manner.

In the following description, reference is made to examples of a system for identifying a potential fault in an electrical circuit comprising a component of a medical imaging system and a switching power supply configured to supply power to the component. In some examples, reference is made to the component being an X-ray tube of a CT imaging system. However, it is to be appreciated that the system described herein may be used to identify potential faults in electrical circuits that include other types of components in a CT imaging system. For instance, the system may be used to identify potential faults in an electrical circuit that includes a gantry rotation mechanism, or a patient table in a CT imaging system. Moreover, it is to be appreciated that the system described herein may be used to identify potential faults in electrical circuits that include other types of components in other types of medical imaging systems. For instance, the system may be used to identify potential faults in an electrical circuit that includes an X-ray tube in a projection X-ray imaging system, or to identify potential faults in an electrical circuit that includes components such as a gantry rotation mechanism, or a patient table, in a projection X-ray imaging system, or an MRI imaging system, or a single photon emission computed tomography "SPECT" imaging system, or a positron emission tomography "PET" imaging system.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

It is also noted that some of the operations that are performed in the computer-implemented methods disclosed herein may be implemented using artificial intelligence techniques. Suitable techniques may include machine learning techniques, as well as deep learning techniques such as using neural networks. For instance, one or more neural networks, may be trained in a supervised, or in some cases unsupervised, manner, to implement the operations performed in the computer-implemented methods disclosed herein.

As mentioned above, there remains a need to improve the identification of faults in the electrical circuits of medical imaging systems.

Fig. 1 is a schematic diagram illustrating an example of a system 100 for identifying a potential fault in an electrical circuit 110, in accordance with some aspects of the present disclosure. Fig. 2 is a flowchart illustrating an example of a method of identifying a potential fault in an electrical circuit, in accordance with some aspects of the present disclosure. The system 100 illustrated in Fig. 1 includes a receiver circuit 150, and an analyser circuit 160. The method illustrated in Fig. 2 represents some of the operations that are performed in the method that are also described as being performed by the receiver circuit 150, and the analyser circuit 160. It is noted that other operations that are described as being performed by the receiver circuit 150, and the analyser circuit 160, may also be performed in the method illustrated in Fig. 2. Moreover, it is noted that the operations that are performed in the method may also be performed by one or more processors, or provided in the form of a computer program product.

With reference to Fig. 1, the system 100 for identifying a potential fault in an electrical circuit 110 comprising a component 120 of a medical imaging system 130 and a switching power supply 140 configured to supply power to the component, comprises:
- a receiver circuit 150 configured to receive radiofrequency, RF, signals generated by the switching power supply 140 during operation of the electrical circuit 110; and
- an analyser circuit 160 configured to determine, based on the received RF signals, an amplitude and/or a phase of one or more harmonic frequencies of a switching frequency of the switching power supply 140, and which one or more harmonic frequencies are generated by the switching power supply 140 in response to a loading of the switching power supply by the component 120; and
- wherein the analyser circuit 160 is further configured to identify the potential fault in the electrical circuit 110 based on the determined amplitude and/or phase of the one or more harmonic frequencies.

An insight exploited by the inventors of the above system is that a switching power supply generates harmonic frequencies, i.e. signals at integer multiples of its switching frequency, that have amplitude, and also phase, values, that are dependent upon the electrical load that is presented to the power supply. In the above system, a load is presented to the switching power supply by the component. The value of the load is determined by the electrical impedance of the component. As a result, the switching power supply generates a set of harmonic frequencies with amplitude and/or phase values that are characteristic of the component.

A further insight of the inventors is that a fault in a component affects its impedance. In the above system, a change in the impedance of the component, changes the loading of the switching power supply. The change in the loading of the switching power supply, changes the amplitude and/or phase values of the harmonic frequencies generated by the switching power supply. Similarly, faults that occur in circuit elements that are elsewhere in the electrical circuit in the above system, also affect their impedance, resulting in changes in the amplitude and/or phase values of the harmonic frequencies generated by the switching power supply. For instance, faults in electrical connectors, or in cables, that couple the switching power supply to the component, and also faults in circuit elements within the switching power supply itself, also result in changes in the amplitude and/or phase values of the harmonic frequencies generated by the switching power supply. Consequently, it is possible to identify potential faults in the electrical circuit that is formed by a switching power supply, and a component of a medical imaging system, based on an amplitude and/or a phase of the one or more harmonic frequencies that are generated by the switching power supply in response to a loading of the switching power supply by the component.

With reference to the example system 100 illustrated in Fig. 1, the system 100 may be used to identify a potential fault in an electrical circuit 110 comprising a component 120 of a medical imaging system 130 and a switching power supply 140 configured to supply power to the component.

In the example illustrated in Fig. 1, the component 120 is an X-ray tube of a CT imaging system. The X-ray tube is mounted to a rotatable gantry 210. During a medical imaging scan, the gantry rotates the X-ray tube 110 and an opposing X-ray detector 220 around an axis of rotation A - A' in order to acquire X-ray attenuation data representing a region of interest. The region of interest may for instance be the chest, or another anatomical region of a patient. The patient may be supported by a patient table 230. The X-ray attenuation data is then reconstructed into a CT image representing the region of interest. X-ray tubes in CT imaging systems such as the one illustrated in Fig. 1 are supplied by a power supply, often referred-to as a "high voltage generator", or simply a "generator". The power supply 140 is used to generate a potential difference between the anode and the cathode of the X-ray tube 120 in order to accelerate electrons from the cathode to the anode in order to generate X-ray radiation. Diagnostic medical imaging systems typically generate X-ray radiation with an energy in the range of 30keV - 120keV. Consequently the potential difference generated by the power supply 140 in the example illustrated in Fig. 1 may be in the range from approximately 30kV to 120kV.

Switching power supplies are often used as the power supply for X-ray tubes due to their high levels of efficiency. An example of a switching power supply that is sometimes used in this application is the so-called Villard cascade, a type of voltage multiplier that is described later with reference to Fig. 3. This type of switching power supply is typically supplied with an alternating current. The alternating current that is supplied to the switching power supply is subsequently supplied to a load via one or more non-linear circuit elements, such as diodes, in order to provide a desired output voltage across the load. In this example, the load is provided by the X-ray tube 120. In this type of switching power supply, the current is inherently switched due to its alternating nature. The switching frequency of the alternating current may be in the range from approximately 1kHz to a few megahertz. Other types of switching power supplies may alternatively be used as the power supply 140, including switching power supplies that are supplied with a DC current. In this case, the switching power supply may include a switching element, e.g. a metal oxide semiconductor field effect transistor "MOSFET" switch, and which switches the current into the load, i.e. the X-ray tube 120, via one or more non-linear circuit elements in order to provide a desired output voltage across the load.

As described above, during operation of the electrical circuit 110 the loading of the switching power supply 140 by the component 120, i.e. the X-ray tube, causes the switching power supply to generate harmonic frequencies with amplitude and/or phase values that are characteristic of the component. As also mentioned above, in general, a switching power supply may be operated at a switching frequency that is in the range from approximately 1kHz to a few megahertz. A significant amount of characteristic information regarding the component may be obtained within approximately the first 100 harmonics, i.e. within the frequency range from approximately a 1kHz to 100MHz.

With continued reference to the system 100 illustrated in Fig. 1, the system 100 includes a receiver circuit 150, and an analyser circuit 160. The receiver circuit 150 is configured to receive radiofrequency, RF, signals generated by the switching power supply 140 during operation of the electrical circuit 110. Thus, the receiver circuit 150 may in general receive RF signals that are in the range from approximately a 1kHz to 100MHz.

The receiver circuit 150 may in general include various amplifiers, filters, analogue-to-digital converters, and other types of circuits for receiving the RF signals generated by the switching power supply 140. For instance, one or more filters may be used to selectively receive RF signals within a desired bandwidth in order to reject interference signals. One or more amplifiers may be used to increase the magnitude of the detected signals. The functionality of some of these circuits may be implemented by hardware, i.e. by discrete, or integrated electronic components, such as analogue-to-digital converters, and field-programmable gate arrays, "FPGAs". The functionality of some of these circuits may alternatively be implemented by one or more processors. For instance, operations such as filtering may be implemented by one or more processors using digital processing techniques.

In general, the receiver circuit 150 may be electrically coupled to the electrical circuit 110, or it may be wirelessly coupled to the electrical circuit 110. In the former case, a resistive, capacitive, or inductive component may be used to electrically couple the electrical circuit 110 to the electrical circuit 110. In the latter case, the receiver circuit 150 may include an antenna for receiving the RF signals generated by the switching power supply 140. The use of wireless coupling, e.g. using an antenna, obviates challenges associated with electrically coupling the receiver circuit to the electrical circuit 110 without influencing the operation of the electrical circuit. The use of wireless coupling also obviates challenges associated with coupling to high voltages that may be present in the electrical circuit 110. Wirelessly-coupling the receiver circuit 150 to the electrical circuit 110 also offers increased the freedom of positioning the receiver circuit in relation to the electrical circuit 110.

In one example, it is contemplated to couple (wirelessly, or electrically) the RF signals from multiple individual electrical circuits into a corresponding number of receiver circuits 150. Each of the individual receiver circuits may be configured to detect a single frequency of interest, or they may be configured to detect multiple frequencies of interest. In another example, it is contemplated to couple (wirelessly, or electrically) the RF signals from multiple electrical circuits into a single receiver circuit 150 using a multiplexer. This enables a single receiver circuit 150 to identify potential faults in multiple different electrical circuits. In examples in which RF signals from multiple frequencies are detected, the RF signals may be mixed down to corresponding relatively lower frequencies and detected by a single receiver circuit 150.

The analyser circuit 160 is configured to determine an amplitude and/or a phase of one or more harmonic frequencies of a switching frequency of the switching power supply 140, based on the received RF signals. In this regard, the analyser circuit may perform various signal analysis operations on the received RF signals in order to determine the amplitude and/or a phase of the one or more harmonic frequencies. For instance, the analyser circuit may demodulate the detected RF signals based on a detection of the switching frequency, or it may apply a (Fast) Fourier transform to the RF signals in order to determine the amplitude and/or the phase of one or more of the harmonic frequencies. As with the receiver circuit 150, some of the functionality of the analyser circuit may be implemented by hardware, and some of the functionality may be implemented by one or more processors, e.g. using digital processing techniques.

The analyser circuit 160 is also configured to identify a potential fault in the electrical circuit 110 based on the determined amplitude and/or phase of the one or more harmonic frequencies. As mentioned above, faults in the component 120, or in circuit elements that are located elsewhere in the electrical circuit, give rise to changes in their electrical impedance. These changes in electrical impedance result in changes in the amplitude and/or phase values of the harmonic frequencies generated by the switching power supply. Consequently, it is possible to identify potential faults in the electrical circuit that is formed by a switching power supply, and a component of a medical imaging system, based on an amplitude and/or a phase of the one or more harmonic frequencies that are generated by the switching power supply in response to a loading of the switching power supply by the component.

The analyser circuit 160 may identify a location of a potential fault in the electrical circuit 110 based on the determined amplitude and/or phase of the one or more harmonic frequencies. For instance, in the example in which the component is an X-ray tube, the analyser circuit may for instance identify a location of the potential fault as being in the X-ray tube, or in the switching power supply 140, or in one or more cables or connectors that supply power from the switching power supply 140 to the X-ray tube. By way of an example, a fault is simulated in the switching power supply 140, and its effect on the harmonic frequencies of the switching power supply is now described with reference to Fig. 3 - Fig. 5.

Fig. 3 is an example of a circuit diagram of an electrical circuit 110 including a switching power supply 140 and a component 120 of a medical imaging system, for use by an electrical circuit simulator to generate simulated amplitude and simulated phase values, in accordance with some aspects of the present disclosure. The electrical circuit 110 illustrated in Fig. 3 may be used by a simulator such as SPICE, pSPICE, and so forth, to determine the characteristic amplitude and/or phase of harmonic frequencies of the switching power supply 140, and also to determine the impact of faults in the electrical circuit 110.

The switching power supply 140 represented in Fig. 3 is a Villard cascade and is supplied by an AC current, as described above. In the circuit illustrated in Fig. 3, the supply of AC current is modelled by the pulsed voltage source V1 at a switching frequency of 50 kHz. The load that is presented to the switching power supply 140 is represented by the component R_load, and corresponds to the impedance between the anode and the cathode of the X-ray tube illustrated in Fig. 1. In order to simulate the effects of defects in the electrical circuit 110, various resistors R1, R_defect2, and R3, have been included in the electrical circuit 110. The values of these resistors may be changed between negligible values, e.g. 1 milliohm, and open-circuit values, e.g. 100 Megohm, and the corresponding simulations used to determine an impact of a short circuit, and an open circuit, respectively. Similar resistors may also be included elsewhere in the electrical circuit at the positions of potential failures in order to simulate the effect of other types of failures.

By way of an example, Fig. 4 is an example of a simulation result obtained by simulating an electrical circuit 110 including a switching power supply and a component of a medical imaging system, in the absence of a simulated defect in an electrical component of the switching power supply, in accordance with some aspects of the present disclosure. The simulation result illustrated in Fig. 4 represents the current delivered by the voltage source V1, i.e. I(V 1), on the ordinate axis, at various frequencies, and which are labelled along the abscissa. The current delivered by the voltage source V1 is indicative of the RF signals emitted by the electrical circuit 110. As may be seen in Fig. 4, the strongest signal occurs at the 50 kHz switching frequency of the switching power supply. Various harmonics of the switching frequency are seen at 50kHz, 100 kHz, 150 kHz, 200 kHz, and so forth. The magnitudes of the signals at the harmonic frequencies illustrated in Fig. 4 may be considered to represent a fingerprint that is characteristic of a correctly functioning electrical circuit 110. Similarly, the phase of the harmonic frequencies (not illustrated in Fig. 4) may also be considered to represent a fingerprint that is characteristic of a correctly functioning electrical circuit 110.

For comparison, Fig. 5 is an example of a simulation result obtained by simulating an electrical circuit 110 including a switching power supply and a component of a medical imaging system, in the presence of a simulated defect in an electrical component of the switching power supply, in accordance with some aspects of the present disclosure. As with the simulation result illustrated in Fig. 4, the simulation result illustrated in Fig. 5 represents the current delivered by the voltage source V1, i.e. I(V1), on the ordinate axis, at various frequencies, and which are labelled along the abscissa. However, the electrical circuit used to obtain the simulation result illustrated in Fig. 5, includes a simulated a short circuit of the diode D3 in Fig. 3. The short circuit is simulated by breaking the circuit at Node A, connecting the cathode of diode D3 to Node B, and connecting the lower terminal of the resistor R1 to Node C. This results in the resistor R3, with value 1 milliohm, being coupled across the diode D3. As compared to the simulation result illustrated in Fig. 4, the magnitudes of the signals at the harmonic frequencies have changed in the simulation result illustrated in Fig. 5. The magnitudes of the signals at the harmonic frequencies illustrated in Fig. 5 may be considered to represent a fingerprint that is characteristic of a failure of the diode D3. Similarly, the phase of the harmonic frequencies (not illustrated in Fig. 5) may also be considered to represent a fingerprint that is characteristic of a failure of the diode D3. In-use, measurements of the harmonic frequencies that are generated by real circuits that correspond to the circuit 110 may be compared with the simulation result illustrated in Fig. 5, in order to identify a potential failure of the diode D3. Similar simulation results may also be generated for simulated failures of other circuit elements in the electrical circuit 110, including the component 120. Thus, more generally, a potential fault in the electrical circuit 110, may be identified based on the amplitude and/or phase of the harmonic frequencies generated by the switching power supply 140.

Various approaches are contemplated for identifying the potential fault in the electrical circuit 110 based on the amplitude and/or phase of the one or more harmonic frequencies that are determined by the analyser circuit.

In one example, the analyser circuit 160 is configured to identify the potential fault in the electrical circuit 110 by comparing the determined amplitude and/or phase of the one or more harmonic frequencies with one or more reference amplitudes and/or one or more reference phase values, respectively.

In this example, the reference amplitudes and/or the one or more reference phase values in this example may be determined based on a simulation 170, 180 of the electrical circuit 110 using an electrical circuit simulator. An example of such a simulation was described above with reference to Fig. 3 - Fig. 5. Reference amplitudes and/or reference phase values that represent the failure of other circuit elements may be determined in a similar manner. The reference amplitude and/or reference phase values for each of the potential failures may be stored, e.g. in a lookup table. Subsequently, a fault may be identified by the analyser circuit by comparing measured the amplitude and/or phase values of the harmonic frequencies with the stored reference amplitude and/or reference phase values for the potential failures, and identifying a closest-match. The comparison may be performed using various mathematical techniques, including for example partial least squares, "PLS", or by using a pattern analysis technique wherein a value of a correlation between the measured values, and the reference values, is evaluated in order to identify a closest match. Alternatively, an artificial intelligence algorithm may be trained to predict a potential fault from measured amplitude and/or phase values of the harmonic frequencies, and wherein the artificial intelligence algorithm is trained using reference amplitude and/or phase values and a ground truth value for their corresponding failure.

In one example, the analyser circuit 160 is configured to determine an amplitude of a plurality of harmonic frequencies of the switching frequency of the switching power supply 140, and the analyser circuit 160 is configured to identify the potential fault in the electrical circuit 110 based on a ratio of the determined amplitudes of the harmonic frequencies. As may be appreciated from the example illustrated in Fig. 1, the ratio between various harmonics may also provide a fingerprint for a failure in a circuit element, such as the diode D3. Identifying the potential fault based on the ratio of the amplitudes of the harmonic frequencies has the advantage of compensating for global changes in the strength of the RF signals that are received from the electrical circuit 110.

In another example, the analyser circuit 160 is configured to determine the amplitude and/or the phase of the one or more harmonic frequencies at each a plurality of points in time. Moreover, the analyser circuit 160 is configured to identify the potential fault in the electrical circuit 110 based on a temporal change in the determined amplitude and/or phase of the one or more harmonic frequencies.

Temporal changes in the amplitude and/or the phase of the one or more harmonic frequencies have been found to be associated with the degradation of circuit elements. Temporal changes may for instance be caused by e.g. degradation-induced changes in the dielectric constant of insulators in the electrical circuit, the build-up of deposits in an X-ray tube, and so forth. In this example, the temporal changes are therefore used as measure of the degradation of circuit elements. The temporal changes may be evaluated by measuring the amplitude and/or phase of the one or more harmonic frequencies at a reference point in time, e.g. during the testing of a component, or during its commissioning. Thereafter, the amplitude and/or phase of the one or more harmonic frequencies may be measured and compared with the measurements at the reference point in time in order to evaluate the change over the intervening time period. The temporal changes may also be used to predict the expected failure of a circuit element by comparing the temporal change with a threshold change value. The threshold change value may be determined empirically from historical monitoring data acquired from an electrical circuit in which a circuit element has failed, or it may be obtained from a simulated failure of a circuit element using the simulation circuit described above.

In some examples, the operation of the electrical circuit 110 may occur contemporaneously with the performance of a medical imaging scan by the medical imaging system 130. In other examples, the operation of the electrical circuit 110 may occur outside of a time of performing a medical imaging scan by the medical imaging system. It is also noted that the component 120 may present a different load to the switching power supply 140 depending on whether or not a medical imaging scan is performed contemporaneously by the medical imaging system. For instance, an X-ray tube typically has a different impedance between its anode and its cathode in each of these situations. Thus, the amplitude and phase values of the harmonic frequencies emitted by the electrical circuit 120 may be dependent upon whether or not a medical imaging scan is being performed contemporaneously. More generally, the amplitude and phase values of the harmonic frequencies emitted by the electrical circuit 120 may be dependent upon a current state of operation of the component. In order to identify a potential fault in such situations, in one example, the analyser circuit 160 may receive status data indicative of a current state of operation of the component 120. The analyser circuit may then identify the potential fault in the electrical circuit based on the status data. In this example, reference values for the amplitude and/or phase values of the harmonic frequencies emitted by the electrical circuit 120 may be obtained by simulating the electrical circuit illustrated in Fig. 3 with different values for the load R_load for each of the different states of operation of the component 120, whereupon the reference values for the relevant state of operation is used to identify the potential fault in the electrical circuit 110.

In a related example, the fingerprint of the harmonic frequencies is used to determine an operational state of the component, or the switching power supply. In this example, the analyser circuit 160 is configured to determine an operational state of the component 120 and/or the switching power supply 140 based on the determined amplitude and/or phase of the one or more harmonic frequencies.

As mentioned above, the operational state of the component impacts its impedance, and this in-turn affects the amplitude and phase values of the harmonic frequencies generated by the switching power supply. Thus, the operational state of the component may be determined based on the determined amplitude and/or phase of the one or more harmonic frequencies. Knowledge of the operational state may be useful in various situations, including for workflow analysis, verifying the safe operation of the medical imaging system, and also in the determination of a subsequent course of action, as described below.

The analyser circuit may perform various operations in response to the identification of a potential fault in the electrical circuit 110. In one example, the analyser circuit 160 is configured to perform one or more of the following operations in response to the identification of the potential fault in the electrical circuit 110:
- generate a notification to a user;
- trigger a change in operation of the component 120; and
- generate a service call to schedule a maintenance operation on the component 120.

In this example, the notification to a user may be provided in the form of a prompt on a display, or as an audio notification. The prompt may for instance indicate the need to verify the operation of a component, or to contact a maintenance provider, and so forth. Alternatively, the identification of the potential fault in the electrical circuit may trigger a change in operation of the component 120. For instance, in the example wherein the component is an X-ray tube, the change in operation of the X-ray tube may for instance include switching the X-ray tube into a de-activated state, or reducing an energy level of the X-ray radiation emitted by the X-ray tube, or reducing an amount of power supplied to the X-ray tube, thereby reducing the risk of damage to the component. The triggering of the change in operation may be performed subject to a user confirmation of a prompt to change the operation of the component 110. Alternatively, the identification of the potential fault in the electrical circuit may automatically generate a service call to schedule a maintenance operation on the component, which again has the effect of reducing the risk of damage to the component.

In a related example, the operations that are performed in response to the identification of a potential fault in the electrical circuit 110, may be performed based on a status data indicative of a current state of operation of the component 120. The status data may be provided by the analyser circuit as described above. In other words, the current status may be determined by the analyser circuit based on the amplitude and/or phase of the one or more harmonic frequencies. The status data may also be generated by other sources. For instance, the status data may be provided by a controller of the medical imaging system, or by one or more sensors configured to monitor the operation of various components of the medical imaging system.

In another example, the analyser circuit 160 is configured to output a replacement time window representing a time period within which the switching power supply 140 and/or the component 120 should be serviced. The replacement time window is determined based on the determined amplitude and/or phase of the one or more harmonic frequencies. The analyser circuit may alternatively output an expected lifetime of the switching power supply and/or the component. The expected lifetime is determined based on the determined amplitude and/or phase of the one or more harmonic frequencies. In this example, the replacement time window, or the expected lifetime may be calculated using various models. As mentioned above, the amplitude and/or phase of the one or more harmonic frequencies is expected to vary as a consequence of degradation of the component, and consequently the time window, or the expected lifetime, may be determined by monitoring the amplitude and/or phase of the one or more harmonic frequencies in relation to reference values for the power supply, or the component, that is close to the end of its expected lifetime. These reference values may be determined empirically, or they may be simulated using the circuit described with reference to Fig. 3 - Fig. 5.

Some examples of models that may be used to calculate the replacement time window, or the expected lifetime, are described in a document by Kang, Z., et al., "Remaining Useful Life (RUL) Prediction of Equipment in Production Lines Using Artificial Neural Networks", Sensors 2021, 21(3), 932. A review of such models is also described in a document by Ahmadzadeh, F., et al., "Remaining useful life estimation", Int. J. Syst. Assur. Eng. Manag. 2014, 5, 461-474. These include physics-based models (e.g. physical model, cumulative damage, hazard rate, proportional hazard rate, nonlinear dynamics), experimental-based models, data-driven models (e.g. neural network, support vector machine, Bayesian network, hidden), hybrid models (e.g. statistical models, Fourier transform with neural network, statistical model with neural network, fuzzy logic with neural network, wavelet transform analysis with a statistical model, dynamic wavelet with neural network).

In another example, the system 100 for identifying a potential fault in an electrical circuit 110 may be included in a medical imaging system. Thus, in this example, the system 100 illustrated in Fig. 1 may be included on, or within a housing of the medical imaging system 130.

In another example, the operations described above with reference to the system 100 illustrated in Fig. 1 are provided in the form of a computer-implemented method. In this example, the computer-implemented method of identifying a potential fault in an electrical circuit 110 comprising a component 120 of a medical imaging system 130 and a switching power supply 140 configured to supply power to the component, includes:
- receiving radiofrequency, RF, signals generated by the switching power supply 140 during operation of the electrical circuit 110; and
- determining, based on the received RF signals, an amplitude and/or a phase of one or more harmonic frequencies of a switching frequency of the switching power supply 140, and which one or more harmonic frequencies are generated by the switching power supply 140 in response to a loading of the switching power supply by the component 120; and
- identifying the potential fault in the electrical circuit 110 based on the determined amplitude and/or phase of the one or more harmonic frequencies.

In another example, the computer-implemented method is provided in the form of a computer program product. In this example, the computer program product comprising instructions which when executed by one or more processors, cause the one or more processors to carry out a method of identifying a potential fault in an electrical circuit 110 comprising a component 120 of a medical imaging system 130 and a switching power supply 140 configured to supply power to the component. The method comprises:
- receiving radiofrequency, RF, signals generated by the switching power supply 140 during operation of the electrical circuit 110; and
- determining, based on the received RF signals, an amplitude and/or a phase of one or more harmonic frequencies of a switching frequency of the switching power supply 140, and which one or more harmonic frequencies are generated by the switching power supply 140 in response to a loading of the switching power supply by the component 120; and
- identifying the potential fault in the electrical circuit 110 based on the determined amplitude and/or phase of the one or more harmonic frequencies.

The above examples are to be understood as illustrative of the present disclosure, and not restrictive. Further examples are also contemplated. For instance, the examples described in relation to the system 100, may also be provided by the computer-implemented method, or by the computer program product, or by a computer-readable storage medium, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A system (100) for identifying a potential fault in an electrical circuit (110) comprising a component (120) of a medical imaging system (130) and a switching power supply (140) configured to supply power to the component, the system comprising:
- a receiver circuit (150) configured to receive radiofrequency, RF, signals generated by the switching power supply (140) during operation of the electrical circuit (110); and
- an analyser circuit (160) configured to determine, based on the received RF signals, an amplitude and/or a phase of one or more harmonic frequencies of a switching frequency of the switching power supply (140), and which one or more harmonic frequencies are generated by the switching power supply (140) in response to a loading of the switching power supply by the component (120); and
- wherein the analyser circuit (160) is further configured to identify the potential fault in the electrical circuit (110) based on the determined amplitude and/or phase of the one or more harmonic frequencies.

2. The system according to claim 1, wherein the analyser circuit (160) is configured to identify the potential fault in the electrical circuit (110) by comparing the determined amplitude and/or phase of the one or more harmonic frequencies with one or more reference amplitudes and/or one or more reference phase values, respectively.

3. The system according to claim 1, wherein the reference amplitudes and/or one or more reference phase values, are determined based on a simulation (170, 180) of the electrical circuit (110) using an electrical circuit simulator.

4. The system according to claim 1, wherein the analyser circuit (160) is configured to determine an amplitude of a plurality of harmonic frequencies of the switching frequency of the switching power supply (140); and
wherein the analyser circuit (160) is configured to identify the potential fault in the electrical circuit (110) based on a ratio of the determined amplitudes of the harmonic frequencies.

5. The system according to claim 1, wherein the analyser circuit (160) is configured to determine the amplitude and/or the phase of the one or more harmonic frequencies at each a plurality of points in time; and
wherein the analyser circuit (160) is configured to identify the potential fault in the electrical circuit (110) based on a temporal change in the determined amplitude and/or phase of the one or more harmonic frequencies.

6. The system according to any previous claim, wherein the operation of the electrical circuit (110) occurs contemporaneously with the performance of a medical imaging scan by the medical imaging system (130).

7. The system according to claim 1, wherein the receiver circuit (150) comprises an antenna configured to receive the RF signals.

8. The system according to any previous claim, wherein the analyser circuit (160) is further configured to perform one or more of the following operations in response to the identification of the potential fault in the electrical circuit (110):
- generate a notification to a user;
- trigger a change in operation of the component (120); and
- generate a service call to schedule a maintenance operation on the component (120).

9. The system according to claim 8, wherein the analyser circuit (160) is further configured receive status data indicative of a current state of operation of the component (120); and wherein the analyser circuit is configured to:
- generate the notification to a user; or
- trigger the change in operation of the component (120); or
- generate the service call to schedule a maintenance operation on the component (120); based on the received status data.

10. The system according to any previous claim, wherein the analyser circuit (160) is further configured to output a replacement time window representing a time period within which the switching power supply (140) and/or the component (120) should be serviced, or an expected lifetime of the switching power supply and/or the component, based on the determined amplitude and/or phase of the one or more harmonic frequencies.

11. The system according to any previous claim, wherein the analyser circuit (160) is further configured to determine an operational state of the component (120) and/or the switching power supply (140) based on the determined amplitude and/or phase of the one or more harmonic frequencies.

12. The system according to any previous claim, wherein the component (120) of the medical imaging system (130) comprises an X-ray tube of a CT imaging system, or an X-ray tube of a projection X-ray imaging system.

13. The system according to claim 12, wherein the switching power supply (140) is configured to supply power to the X-ray tube via one or more cables and/or one or more connectors, and wherein the analyser circuit (160) is configured to identify a location of the potential fault as being in one or more of the following locations in the electrical circuit (110): in the X-ray tube, in the switching power supply (140), in the one or more cables, and in the one or more connectors.

14. A medical imaging system (130) comprising the system according to any one of claims 1 - 13.

15. A computer-implemented method of identifying a potential fault in an electrical circuit (110) comprising a component (120)of a medical imaging system (130) and a switching power supply (140) configured to supply power to the component, the method comprising:
- receiving radiofrequency, RF, signals generated by the switching power supply (140) during operation of the electrical circuit (110); and
- determining, based on the received RF signals, an amplitude and/or a phase of one or more harmonic frequencies of a switching frequency of the switching power supply (140), and which one or more harmonic frequencies are generated by the switching power supply (140) in response to a loading of the switching power supply by the component (120); and
- identifying the potential fault in the electrical circuit (110) based on the determined amplitude and/or phase of the one or more harmonic frequencies.
